# EUROPEAN PATENT SPECIFICATION

(11) **EP 1 075 270 B1**
(45) Date of publication and mention of the grant of the patent: **15.06.2005**
(21) Application number: 99916409.8
(22) Date of filing: 06.04.1999
(51) Int. Cl.: C07H 21/04, C07K 16/00, G01N 33/53, A61K 38/00, A61K 48/00, A61K 38/04, C07K 5/00, C07K 7/04

(54) **SHORT PEPTIDE FOR TREATMENT OF NEUROLOGICAL DEGENERATIVE DISEASES**
KURZES PEPTID ZUR BEHANDLUNG NEURODEGENERATIVER ERKRANKUNGEN
PEPTIDE COURT DESTINE AU TRAITEMENT DES MALADIES NEURODEGENERATIVES

(30) Priority: 06.04.1998 US 80836 P
(43) Date of publication of application: 14.02.2001
(73) Proprietor: Advanced Immunit, Inc., Stony Brook, NY 11790 (US)
(72) Inventor: PERT, Candace, Potomac, MD 20854 (US); RUFF, Michael, Potomac, MD 20854 (US)
(74) Representative: Larsen, Hans Ole
(86) International application number: PCT/US1999/007514
(87) International publication number: WO 1999/051254

(56) References cited:
- US-A- 5 276 016
- US-A- 5 567 682
- HAUSER J ET AL: "Chemokines prevent neuronal cell death associated with the HIV envelope protein (gp120)." SOCIETY FOR NEUROSCIENCE ABSTRACTS, vol. 23, no. 1-2, 1997, page 993 XP009015668 27th Annual Meeting of the Society for Neuroscience, Part 1;New Orleans, Louisiana, USA; October 25-30, 1997 ISSN: 0190-5295
- COCCHI F ET AL: "IDENTIFICATION OF RANTES, MIP-1ALPHA, AND MIP-1BETA AS THE MAJOR HIV-SUPPRESSIVE FACTORS PRODUCED BY CD8+ T CELLS" SCIENCE, AMERICAN ASSOCIATION FOR THE ADVANCEMENT OF SCIENCE,, US, vol. 270, 15 December 1995 (1995-12-15), pages 1811-1815, XP000616644 ISSN: 0036-8075
- MEUCCI OLIMPIA ET AL: "Chemokines regulate hippocampal neuronal signaling and gp120 neurotoxicity." PROCEEDINGS OF THE NATIONAL ACADEMY OF SCIENCES OF THE UNITED STATES, vol. 95, no. 24, 24 November 1998 (1998-11-24), pages 14500-14505, XP002235894 Nov. 24, 1998 ISSN: 0027-8424
- RUFFING NANCY ET AL: "CCR5 has an expressed ligand-binding repertoire and is the primary receptor used by MCP-2 on activated T cells." CELLULAR IMMUNOLOGY, vol. 189, no. 2, 1 November 1998 (1998-11-01), pages 160-168, XP002235895 ISSN: 0008-8749
- BRENNEMAN D E ET AL: "NEURONAL CELL KILLING BY THE ENVELOPE PROTEIN OF HIV AND ITS PREVENTION BY VASOACTIVE INTESTINAL PEPTIDE" NATURE, MACMILLAN JOURNALS LTD. LONDON, GB, vol. 335, no. 6191, 13 October 1988 (1988-10-13), pages 639-642, XP000069014 ISSN: 0028-0836
- BRENNEMAN DOUGLAS E ET AL: "VIP and D-ala-peptide T-amide release chemokines which prevent HIV-1 gp120-induced neuronal death." BRAIN RESEARCH, vol. 838, no. 1-2, 14 August 1999 (1999-08-14), pages 27-36, XP002235896 ISSN: 0006-8993
- BRENNEMAN D E ET AL: "CHEMOKINES RELEASED FROM ASTROGLIA BY VASOACTIVE INTESTINAL PEPTIDE MECHANISM OF NEUROPROTECTION FROM HIV ENVELOPE PROTEIN TOXICITY" CUTANEOUS NEUROIMMUNOMODULATION: THE PROOPIOMELANOCORTIN SYSTEM, XX, XX, vol. 921, December 2000 (2000-12), pages 109-114, XP009008293

## Description

### TECHNICAL FIELD

This invention is directed to synthetically produced short peptide sequences which inhibit HIV-1 gpl20 induced neuronal cell death, for use in preventing neurological deterioration in patients suffering from AIDS as well as other neurological degenerative diseases.

### BACKGROUND

Among the symptoms and conditions associated with HIV infection (AIDS) are specific neurological conditions which can be termed "neuro-AIDS".

Neuro-AIDS, whose incidence and severity appears to be increasing, can manifest itself in many forms including encephalopathies, neuropathies, memory loss, dementia, depression, psychosis and opportunistic infections. One explanation for AIDS associated neuropathologies, which can include infiltration of infected immune cells, white matter aberrations, reduced dendritic and axonal arborization, and neuronal loss is that dissociated HIV envelop protein, gpl20, which has been shown to be secreted abundantly by infected macrophages and is present in plasma and CSF, contributes to pathogenesis via receptor-mediated interactions with various shared cell surface receptors on brain and immune cells.

There is growing evidence that neurotoxicity and infectivity associated with HIV have distinctive attributes suggesting divergence of mechanism. In particular, HIV infection does not occur in rodents and does not require signaling, while the biological activities associated with the envelope protein can be demonstrated in both human and rodent cells and requires signaling. The neurotoxic action of HIV-1 envelope protein gpl20 is potent and requires the presence of glial cells, which may then secrete neurotoxic products or cytokines. In rodents, intraventriculary administered gpl20 produces endocrine abnormalities.

The neuropeptide vasoactive intestinal peptide (VIP), as well as homologous short (5-8 residues) peptides derived from the gpl20 V2 region derived peptides (8-10) are inhibitors of gpl20 neurotoxicity. In neonatal rats, delayed behavioral milestones and abnormal neuronal dearborization produced by administration of nanogram quantities of gpl20 are also prevented by VIP (II) and gpl20 V2-region derived peptide T ("DAPTA"). In the same study, toxic fragments of gpl20 were recovered from treated animals, suggesting that some of the neural damage is attributable to proteolytic products of the HIV envelope.

Alzheimers' Disease or dementia is believed to be caused by the deterioration of the cholinergic neurons in the basal forebrain. VIP is co-localized with cholinergic neurons in the basal forebrain and is believed to maintain neuronal survival. In a proposed secondary phase of Alzheimers' disease, endogenous neurons of the cortex of various different chemical types degenerate following deprivation of their vasoactive intestinal polypeptide neuronal growth factor once contained in the cholinergic endings.

In U.S. Patent No. 5,567,682, short chain peptides, specifically peptide T and related analogs, are described for treating the symptoms of Alzheimers' disease by reducing or halting a loss of neurons. Similarly, these peptides are described as being useful in inhibiting HIV-1 binding to T4 cell receptors (U.S. Patent No. 5,276,016).

Recent discoveries show that HIV gpl20 uses a number of chemokine co-receptors, in conjunction with CD4, to allow viral entry of target cells. Moreover, various gpl20's can block binding of specific chemokine ligands with the CCR5 receptor. Chemokine receptors, first characterized on activated immune cells, have been shown to also be present on cerebellar neuronal processes, differentiated human neuronal lines, and both microglial cells and astrocytes in human brain cultures. Thus the inventors sought to identify novel short chemokine peptides which would be antagonists of gpl20-mediated neurotoxicity and resultant neuronal degeneration and thereby provide therapeutic benefits in patients suffering from HIV infection, or other inflammatory neurological diseases such as multiple sclerosis, tropical spastic paraparesis, and Alzheimers, to cite some.

### SUMMARY OF THE INVENTION

It is an object of the present invention to provide peptides and a method for treating diseases having symptoms caused by neuronal cell death caused by HIV, MS, Alzheimers' Disease, PML, and Tropical Spastic Paraparesis, among others.

It is another object to provide a pharmaceutical composition having a peptide as an active agent for reducing or inhibiting neuronal cell loss.

These and other objects of the present invention are achieved by a peptide of the formula

Leu-Glu-Ser-Tyr-Thr .

Moreover, the application discloses the following peptide :

Ile-Lys-Glu-Tyr-Phe-Thr-Ser

A method for treating the symptoms associated with neuronal cell death in a person caused by a neurological degenerative disease comprises administering a therapeutically effective amount of a peptide of the formula

Leu-Glu-Ser-Tyr-Thr

The invention comprises a peptide of the formula Leu-Glu-Ser-Tyr-Thr or a physiologically acceptable salt thereof. A pharmaceutical composition comprising as an active ingredient at least one peptide of the formula Leu-Glu-Ser-Tyr-Thr or a pharmaceutically acceptable salt thereof, for treating the symptoms caused by neuronal cell loss. The pharmaceutical composition can further comprise a pharmaceutically acceptable carrier.

The invention also includes a method for treating the symptoms caused by a loss of neurons comprising administering to a person suffering from a disease causing neuronal cell loss a therapeutically effective amount of a people of formula Leu-Glu-Ser-Tyr-Thr or a pharmaceutically acceptable salt thereof. According to the method, the peptide is administered by oral, intranasal, buccal, parenteral, topical or rectal administration.

### BRIEF DESCRIPTION OF THE DRAWINGS

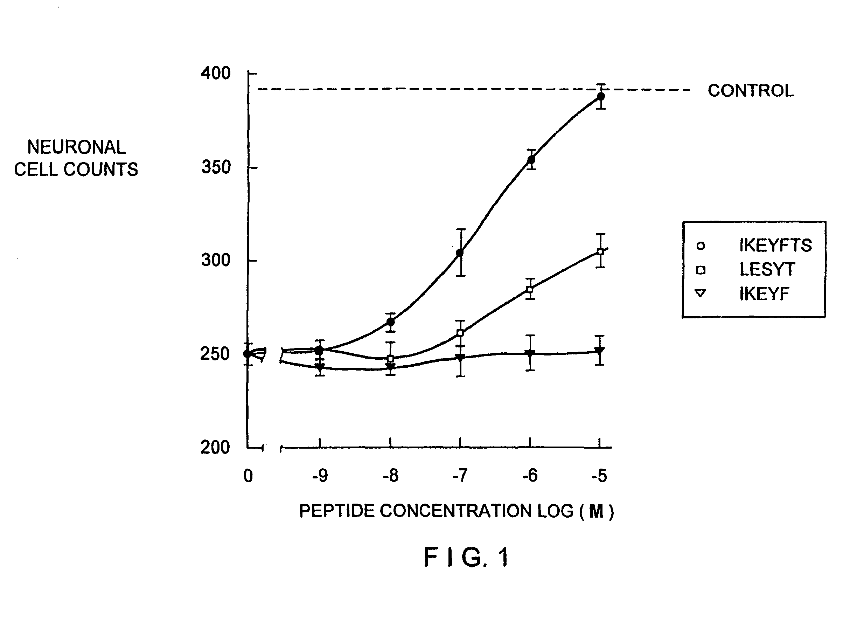

Figure 1 shows neuronal cell survival with reference to peptide concentration.

### DETAILED DESCRIPTION OF THE INVENTION

*Materials:* A gpl20 isolate RFII was obtained from Dr. P.Nara, NCI, NIH. All of the gpl20's are purified, >95% homogeneous and previously tested for neurotoxicity with cultured neurons and were used at IpM final concentration in cultures of neurons. Peptides of the formula Ile-Lys-Glu-Tyr-Phe-Thr-Ser, Ile-Lys-Glu-Tyr-Phe, and Leu-Glu-Ser-Tyr-Thr were obtained from Peninsula Labs, CA. and synthesized by solid-phase Merrifield methods and purified to greater than 95% homogeneity by two HPLC methods and structure confirmed by MASS Spectroscopy Analysis.

*Neuronal cell culture:* Dissociated hippocampal cultures are prepared from neonatal (day 2) rat cortex and hippocampus by known methods. Sterilely dissected brain tissue is minced and treated with 0.125% trypsin for 30 minutes then gently triturated with fire-polished Pasteur pipes and plated in six-well trays (35mm²) at low density (50,000 cells/35mm² dish) upon confluent layers of astrocytes in medium containing D-MEM, penicillin (25 U/ml), streptomycin (25 mg/ml), D-glucose (0.6%), and 10% heat-inactivated fetal calf serum (HyClone Laboratories), supplemented with insulin, transferrin, selenium, corticosterone, and triiodothyronine. Medium is changed after 3 days. At 6 days, half the medium is exchanged for fresh medium. The astrocyte feeder layers were prepared from the cortexes of neonatal rats following dissection and trituration. Plating was 2.5 X10² cells per well. Feeder layers were grown in Eagles's minimal essential medium (Formula No. 82-0234DJ, Gibco) with 10% fetal calf serum until confluent (7-14 days). With this medium the feeder cultures were free of neurons and consisted of flat cells that were stained by antibodies to glial fibrillary acidic protein, a standard immunocytochemical marker for astroglia. When hippocampal or cortical cells were added to the confluent feeder layer, the medium was changed to the following composition: 5% horse serum and MEM supplement with defined medium components. The hippocampal or cortical cultures were treated with 5'-fluoro-2'-deoxyuridine (15ug/ml plus uridine, 35ug/ml) to suppress the overgrowth of background cells and allow the establishment of neurons. The neurons in these cultures are post-mitotic. The neuronal cultures were allowed to grow for 1 week prior to the beginning of the experimental period. Before treating the cultures, a complete change of medium was given.

*Neuronal survival assay:* GP120's, with or without added peptides, were diluted in phosphate buffered saline and added to the cultures, which were treated only once for a four day period. At termination, cultures were fixed with glutaraldehyde as previously described. At the end of the test period, neuronal survival was assessed by immunohistochemical detection of neuron-specific enolase positive cells (neurons). Cultures were counted in a blinded fashion without knowledge of sample treatment in 40 fields at pre-determined coordinate locations. The total area counted is 50 mm². Each value reported is the mean ± the standard error of 3-4 determinations. Control (saline treated) wells from these cultures have 395 ± 20 neurons. Statistical comparisons between experimental and control culture neuronal cell counts are via analysis of variance with the Student-Newman-Kuels multiple comparison of means test.

### Results

### Effects of Short Chemokine Derived Peptides on GP120-Mediated Neurotoxicity

When the peptides were added to primary cultures of mixed rat neurons/glia, together with 1 pM gpl20 (RF isolate), which by itself killed about half of the neurons in the dish ( Fig. 1), neuronal death could be inhibited. In a dose-dependent fashion, significant increases in cell counts were observed from cultures treated with gp120 alone, with IKEYFTS and LESYT preventing neuronal loss caused by gpl20. The peptide IKEYFTS had an EC50 of IOO nM and was fully protective at IOuM, while LESYT was partially protective at IOuM. Specificity is shown in that the shorter pentapeptide IKEYF was inactive. The dotted line in Figure 1 represents the mean number of neurons in control cultures.

The peptides also supported the viability of neurons in the absence of added gpl20 and thus acted as survival factors. The results thereby identify novel, short chemokine related peptides which have significant neuroprotective activity against gpl20 neurotoxicity as well as promote neuronal survival and which therefore may be treatments for AIDS and other neurodegenerative diseases which includes, but is not limited to conditions like Alzheimers, multiple sclerosis, tropical paraparesis, PML and neuropathies of various etiologies including diseases resulting from or relating to HTLV-1 infection, to cite some examples.

The peptides may be administered in suitable carriers by various routes including oral, buccal, iv, rectal, nasal, with effective doses from 0.01 mg to 1000 mgs per day, preferably from 0.2 to 10 mg per day for a 70 kg person.

The active compounds of the invention may exist as physiologically acceptable salts of the peptides.

The compounds of the invention may be beneficially modified by methods known to enhance passage of molecules across the blood-brain barrier. Acetylation has proven to be especially useful for enhancing binding activity of the peptide. The terminal amino and carboxy sites are particularly preferred sites for modification.

The peptides of this invention may also be modified in a constraining conformation to provide improved stability and oral availability.

Unless otherwise indicated the amino acids are, of course, the natural form of L-stereoisomers.

The peptides that are to be administered intranasally in accordance with the invention may be produced by conventional methods of peptide synthesis. Both solid phase and liquid phase methods, as well as other methods e.g., enzymatic methods, may be used. We have found the solid phase method of Merrifield to be particularly convenient. In this process the peptide is synthesized in a stepwise manner while the carboxy end of the chain is covalently attached to an insoluble support. During the intermediate synthetic stages the peptide remains in the solid phase and therefore can conveniently manipulated. The solid support is a chloromethylated styrene-divinylbenzene copolymer.

As an aspect of the invention, therefore, we provide a pharmaceutical compositions comprising a peptide compound of the invention in association with pharmaceutically acceptable carrier or excipient, adapted for use in human or veterinary medicine. Such compositions may be presented for use in a conventional manner in admixture with one or more physiologically acceptable carriers of excipient. The compositions may optionally further contain one or more other therapeutic agents which may, if desired, be a different antiviral agent.

Thus, the peptides according to the invention may be formulated for oral, intranasal, buccal, parenteral, topical or rectal administration.

In particular, the peptides according to the invention may be formulated for injection or for infusion and may be presented in unit dose form in ampoules or in multidose containers with an added preservative. The compositions may take such forms as suspensions, solutions, or emulsions in oily or aqueous vehicles, and may contain formulatory agents such as suspending, stabilizing and/or dispersing agents. Alternatively, the active ingredient may be in powder form for constitution with a suitable vehicle, e.g., sterile, pyrogen-free water, before use. In a particularly preferred embodiment, the active ingredient may be administered intranasally, preferably in more than one daily application.

The pharmaceutical compositions according to the invention may also contain other active ingredients such as antimicrobial agents, or preservatives.

A further aspect of this invention relates to vaccine preparations containing a peptide according to the invention, to provide protection against viral infection. The vaccine will contain an effective immunogenic amount of peptide, e.g. 1µg to 20 mg/kg of host, optionally conjugated to a protein such as human serum albumin, in a suitable vehicle, e.g. sterile water, saline or buffered saline. Adjuvants may be employed, such as aluminum hydroxide gel. Administration may be by injection, e.g. intramuscularly, interperitoneally, subcutaneously or intravenously. Administration may take place once or at a plurality of times, e.g. at 1-4 week intervals.

Antigenic sequences from crab as well as proteins from other invertebrates can also be added to the peptides of the invention to promote antigenicity.

## Claims

1. A peptide of the formula Leu-Glu-Ser-Tyr-Thr or a physiologically acceptable salt thereof.

2. A pharmaceutical composition comprising as an active ingredient at least one peptide of the formula Leu-Glu-Ser-Tyr-Thr or a pharmaceutically acceptable salt thereof, for treating the symptoms caused by neuronal cell loss.

3. The pharmaceutical composition of claim 2 further comprising a pharmaceutically acceptable carrier.

4. Use of a peptide of the formula Leu-Glu-Ser-Tyr-Thr for the manufacture of a medicament for treating AIDS and other neurodegenerative diseases.

5. Use of a peptide according to claim 4, wherein other neurodegenerative diseases are selected from the group consisting of Alzheimers, multiple sclerosis, tropical paraparesis, PLM and neuropathies of various etiologies including diseases resulting from or relating to HTLV-1 infection.

6. Use of a peptide according to any of claims 1-5, wherein the peptide is in a form to be administered by oral, intranasal, buccal, parenteral, topical or rectal administration.

## Patentansprüche

1. Peptid der Formel Leu-Glu-Ser-Tyr-Thr oder ein physiologisch annehmbares Salz davon.

2. Pharmazeutische Zusammensetzung umfassend als wirksamen Inhaltsstoff mindestens ein Peptid der Formel Leu-Glu-Ser-Tyr-Thr oder ein pharmazeutisch annehmbares Salz davon, zur Behandlung von durch Verlust von neuronalen Zellen hervorgerufen Symptomen.

3. Pharmazeutische Zusammensetzung gemäß Anspruch 2, weiterhin umfassend einen pharmazeutisch annehmbaren Träger.

4. Verwendung eines Peptids der Formel Leu-Glu-Ser-Tyr-Thr zur Herstellung eines Medikaments zur Behandlung von Aids und anderen neurodegenerativen Erkrankungen.

5. Verwendung eines Peptids gemäß Anspruch 4, wobei die anderen neurodegenerativen Erkrankungen ausgewählt sind aus der Gruppe bestehend aus Alzheimer, Multiple Sklerose, Tropen-Paraparese, PLM und Neuropathien verschiedener Ätiologie einschließlich Erkrankungen hervorgerufen durch oder im Zusammenhang mit HTLV-1-Infektionen.

6. Verwendung eines Peptids gemäß einem der Ansprüche 1 bis 5, wobei das Peptid in einer Verabreichungsform vorliegt, dass es oral, intranasal, bukkal, parentaral, topisch oder rektal verabreicht werden kann.

## Revendications

1. Peptide de formule Leu-Glu-Ser-Tyr-Thr ou un sel physiologiquement acceptable de celui-ci.

2. Composition pharmaceutique comprenant comme ingrédient actif au moins un peptide de formule Leu-Glu-Ser-Tyr-Thr ou un sel pharmaceutiquement acceptable de celui-ci, pour traiter les symptômes provoqués par une perte cellulaire neuronale.

3. Composition pharmaceutique selon la revendication 2, comprenant en outre un support pharmaceutiquement acceptable.

4. Utilisation d'un peptide de formule Leu-Glu-Ser-Tyr-Thr pour la fabrication d'un médicament pour traiter le SIDA et d'autres maladies neurodégénératives.

5. Utilisation d'un peptide selon la revendication 4, dans laquelle les autres maladies neurodégénératives sont choisies dans le groupe consistant en la maladie d'Alzheimer, la sclérose multiple, la paraparèsie tropicale, PLM et des neuropathies de diverses étiologies y compris des maladies résultant de l'infection HTLV-1 ou relatives à celle-ci.

6. Utilisation d'un peptide selon l'une des revendications 1 à 5, dans laquelle le peptide est sous une forme à administrer par administration orale, intranasale, buccale, parentérale, topique ou rectale.
